# EUROPÄISCHE PATENTSCHRIFT

(11) **EP 1 867 297 B1**
(45) Veröffentlichungstag und Bekanntmachung des Hinweises auf die Patenterteilung: **11.03.2009**
(21) Anmeldenummer: 06012358.5
(22) Anmeldetag: 16.06.2006
(51) Int. Cl.: A61C 1/08

(54) **Set beinhaltend mehrere Bohrstopphülsen und einen Montageblock**
set containing several drilling stop guides and an assembling aid
set contenant plusieurs butées et un block de montage

(43) Veröffentlichungstag der Anmeldung: 19.12.2007
(73) Patentinhaber: Straumann Holding AG, 4002 Basel (CH)
(72) Erfinder: Hirsch, Erika, 4414 Füllinsdorf (CH); Näf, Patrick, 8057 Zürich (CH)
(74) Vertreter: Schaad, Balass, Menzl & Partner AG

(56) Entgegenhaltungen:
- EP-A2- 0 373 111
- EP-A2- 0 385 059
- WO-A-00/25695
- WO-A-20/06062613
- WO-A2-00/74585
- DE-U1- 9 106 606
- FR-A- 2 878 429
- US-A- 74 466
- US-A- 2 700 905
- US-A- 5 788 488
- US-A1- 2005 106 531
- US-A1- 2006 004 372
- US-B1- 6 514 258

## Beschreibung

Die vorliegende Erfindung betrifft ein Set beinhaltend mehrere Bohrstopphülsen und einen Montageblock gemäss Anspruch 1.

Eine Bohrstopphülse dieser Art sowie eine Dentalbohrvorrichtung umfassend einen Dentalbohrer und eine Bohrstopphülse sind aus US 6,514,258 B1 bekannt. Diese Druckschrift offenbart einen Dentalbohrer, der ein Bohrende und einen zylinderförmigen Schaftteil aufweist. An einem dem Bohrende zugewandten Endbereich des Schaftteils ist dieser mit einer manschettenartigen Verdickung versehen, die einen grösseren Durchmesser aufweist, als der Bohrdurchmesser des Dentalbohrers. Die Bohrstopphülse weist an einer ihrer Stirnseiten eine Anschlagfläche und angrenzend an die andere Stirnseite einen Klemmbereich auf, der dazu bestimmt ist, die Verdickung zu umgreifen. Die Bohrstopphülse weist im Klemmbereich den grössten Aussendurchmesser auf, welcher durch mehrere federnde Klemmfinger ausgebildet ist. Diese sind durch in Längsrichtung der Bohrstopphülse verlaufende, stirnseitig offene Spalten ausgebildet. Die Bohrstopphülse ist vom Bohrende her auf den Dentalbohrer aufschiebbar, wodurch der Klemmbereich schnappverschlussartig mit der Verdickung zum Zusammenwirken kommt.

Um ein Loch einer bestimmten Bohrtiefe zu bohren, offenbart US 6,514,258 B1 verschieden lange Bohrstopphülsen, die auf den Dentalbohrer gegebener Länge aufsetzbar sind. Durch den Abstand des Bohrendes zur Anschlagfläche der aufgesetzten Bohrstopphülse ist jeweils die Bohrtiefe der Dentalbohrvorrichtung festgelegt. Weiter offenbart diese Druckschrift, dass Dentalbohrer mit zwei verschiedenen Bohrdurchmessern zur Anwendung gelangen können. Als Material für die Bohrstopphülse ist ausgeführt, dass starre, steife Materialien, wie Metall oder Plastik, zur Anwendung gelangen können.

Als nachteilig an der Bohrstopphülse gemäss diesem Stand der Technik erweist sich, dass die Sicht auf das Bohrloch durch die Bohrstopphülse teilweise verdeckt ist, insbesondere kurz bevor die Anschlagfläche an den Knochen beziehungsweise an das das Bohrloch umgebende Gewebe oder Material ansteht. Ebenfalls als nachteilig erweist sich, dass im Klemmbereich mehrere fingerartige Klemmelemente ausgebildet sind, wodurch die Fertigung aufwendig und somit teuer ist. Ebenso als nachteilig erweist sich, dass die Bohrstopphülse im Klemmbereich den grössten Aussendurchmesser aufweist, wodurch die Sicht ebenfalls behindert wird.

US 2006/0004372 offenbart auch eine Dentalvorrichtung umfassend einen Dentalbohrer und eine Bohrstopphülse WO 2006/062613 offenbart ein Set wobei kleine Teile in Blistern gehalten sind.

Aufgabe der vorliegenden Erfindung ist es, eine Bohrstopphülse und eine Dentalbohrvorrichtung bereitzustellen, die die oben genannten Nachteile nicht aufweisen. Weiter soll eine die Handhabung der Bohrstopphülsen erleichternde Verpackung bereitgestellt werden.

Diese Aufgaben werden erfindungsgemäss mit einem Set gemäss Anspruch 1.

Durch die transparente oder transluszente Ausbildung der Bohrstopphülse, kann die Sicht auf das Bohrloch entscheidend verbessert werden, ohne die Wandstärke der Bohrstopphülse und somit die Grösse der Anschlagsfläche zu verringern.

Gemäss einer bevorzugten Ausführungsform der Bohrstopphülse weist diese in einem hohlzylinderförmigen Bereich eine radiale Durchgangsöffnung auf. Diese ermöglicht, dass aus dem Bohrloch gefördertes Gewebe und Knochenmaterial vom radial Inneren der Bohrstopphülse auf die Aussenseite der Bohrstopphülse gefördert werden kann. Weiter dient die radiale Durchgangsöffnung der Sicherstellung der Kühlung des Dentalbohrers im Inneren der Bohrstopphülse.

Gemäss einer bevorzugten Ausführungsform weist der hohlzylinderförmige Bereich eine grössere Wandstärke auf als der Befestigungsbereich, und der Innendurchmesser des Befestigungsbereichs verjüngt sich in Richtung des freien Endes. Dadurch lässt sich ebenfalls erreichen, dass die Sicht auf das Bohrloch verbessert ist.

Gemäss einer bevorzugten Ausführungsform der Bohrstopphülse ist ein Mittel zum Fixieren der Bohrstopphülse in einem der Anschlagsfläche gegenüberliegenden Endbereich angeordnet und durch eine Lippe ausgebildet, die dazu bestimmt ist, mit einem Haltebereich am Dentalbohrer zusammen zu wirken. Durch die ununterbrochene, umlaufende und radial elastisch deformierbare Lippe ist der Befestigungsbereich der Bohrstopphülse auf einfachste Weise stabil ausbildbar und somit kostengünstig realisierbar.

Durch die erfindungsgemässe Dentalbohrvorrichtung ist eine sehr einfache und dadurch kostengünstige Ausbildung des Dentalbohrers und der Bohrstopphülse realisierbar.

Gemäss einer bevorzugten Ausführungsform ist die Bohrstopphülse derart elastisch deformierbar ausgebildet, dass sie über den Schaftteil auf den Dentalbohrer aufsetzbar ist. Durch diese elastische Deformierbarkeit ist die Montage der Bohrstopphülse auf den Dentalbohrer auf einfachste Weise realisierbar, ohne Gefahr der Beschädigung der Bohrstopphülse durch die Schneiden des Dentalbohrers.

Gemäss einer bevorzugten Ausführungsform ist die Bohrstopphülse wie auch der Dentalbohrer mit einer Kodierung, insbesondere einer Farbkodierung versehen. Dadurch ist es beispielsweise auf einfachste Weise möglich, eine zum Bohrdurchmesser des Dentalbohrers passende Bohrstopphülse zu kennzeichnen.

Durch das erfindungsgemässe Set ist eine sterile Bereitstellung der bei einer Operation anzuwendenden Bohrstopphülsen auf einfachste Weise erreichbar. Vor der Operation wird ein Blister mit Bohrstopphülsen der gewünschten Länge ausgewählt, wodurch in Kombination mit dazu passenden Dentalbohrern die gewünschte Bohrtiefe festgelegt ist. Bei der Operation wird zunächst ein Dentalbohrer mit dem kleinsten Bohrdurchmesser zusammen mit der zu diesem passenden Bohrstopphülse verwendet. Das resultierende Bohrloch wird - falls notwendig - mit dem Dentalbohrer mit dem nächst grösseren Bohrdurchmesser aufgebohrt, wobei wiederum auf den Dentalbohrer die zu diesem passende Bohrstopphülse aufgesetzt ist. Falls notwendig kann das Aufbohren mit Dentalbohrern grösserer Bohrdurchmesser und den dazu passenden Bohrstopphülsen fortgesetzt werden.

Weitere bevorzugte Ausbildungsformen der erfindungsgemässen Bohrstopphülse, der erfindungsgemässen Dentalbohrvorrichtung und des erfindungsgemässen Sets sind in den weiteren abhängigen Ansprüchen angegeben.

Weitere besondere Vorteile und Wirkungsweisen ergeben sich aus der Detailbeschreibung und der Zeichnung.

Im Folgenden wird die Erfindung anhand eines in der Zeichnung dargestellten Ausführungsbeispiels näher erläutert. Es zeigen rein schematisch:
- Fig. 1: in Seitenansicht einen Dentalbohrer, der an seinem Schaftteil eine Farbkodierung und an seinem Schneidteil kreisringartige Bohrtiefenmarkierungen aufweist;
- Fig. 2: in Seitenansicht eine erfindungsgemässe Dentalbohrvorrichtung mit einem Dentalbohrer und einer auf den Dentalbohrer aufgesetzten Bohrstopphülse, wobei die Bohrstopphülse teilweise geschnitten dargestellt ist;
- Fig. 3: in perspektivischer Darstellung die erfindungsgemässe Dentalbohrvorrichtung, bei welcher auf den in Fig. 1 gezeigten Dentalbohrer eine erfindungsgemässe Bohrstopphülse aufgesetzt ist;
- Fig. 4: in Seitenansicht den durch "X" gekennzeichneten Detailausschnitt der Fig. 1, der einen Halteabschnitt zeigt;
- Fig. 5: in Seitenansicht den durch "X" gekennzeichneten Detailausschnitt der Fig. 2, der den Halteabschnitt zeigt;
- Fig. 6: in Schnittdarstellung eine erste Ausführungsform der Bohrstopphülse;
- Fig. 7: in Schnittdarstellung den durch "X" gekennzeichneten Detailausschnitt der Fig. 6;
- Fig. 8: in perspektivischer Darstellung die Bohrstopphülse gemäss Fig. 6;
- Fig. 9: in Schnittdarstellung eine Ausführungsform der Bohrstopphülse, welche im Vergleich zu der in Fig. 6 bis 8 gezeigten Bohrstopphülse für einen Dentalbohrer mit einem grösseren Bohrdurchmesser gefertigt ist und dieselbe Länge wie diese aufweist;
- Fig. 10: in Schnittdarstellung den durch "X" gekennzeichneten Detailausschnitt der Fig. 9;
- Fig. 11: in Schnittdarstellung eine Ausführungsform der Bohrstopphülse, welche im Vergleich zu der in Fig. 9 und 10 gezeigten Bohrstopphülse für einen Dentalbohrer mit einem grösseren Bohrdurchmesser gefertigt ist und dieselbe Länge wie diese aufweist;
- Fig. 12: in Schnittdarstellung den durch "X" gekennzeichneten Detailausschnitt der Fig. 11;
- Fig. 13: in Schnittdarstellung eine Ausführungsform der Bohrstopphülse, welche im Vergleich zu der in Fig. 11 und 12 gezeigten Bohrstopphülse für einen Dentalbohrer mit einem wiederum grösseren Bohrdurchmesser gefertigt ist und dieselbe Länge wie diese aufweist;
- Fig. 14: in Schnittdarstellung den durch "X" gekennzeichneten Detailausschnitt der Fig. 14;
- Fig. 15: in Draufsicht eine Setbox mit sechs Haltevorrichtungen für Blister und einer Haltevertiefung für eine Montagehilfe, wobei in zwei dieser Haltevorrichtungen ein beziehungsweise drei Blister eingesetzt sind;
- Fig. 16: im Schnitt, entlang der in Fig. 15 angegebenen Schnittebene A-A, die dort gezeigte Setbox mit eingesetzten Blistern;
- Fig. 17: im Schnitt, entlang der in Fig. 15 angegebenen Schnittebene B-B, die dort gezeigte Setbox mit eingesetzten Blistern;
- Fig. 18: in Draufsicht einen Blister mit insgesamt vier Aufnahmevertiefungen für je eine Bohrstopphülse, wobei alle Bohrstopphülsen dieselbe Länge aufweisen und jede der Bohrstopphülsen für einen Dentalbohrer unterschiedlichen Bohrdurchmessers ausgelegt ist;
- Fig. 19: in Schnittdarstellung gemäss der in Fig. 18 eingezeichneten Schnittebene A-A den Blister;
- Fig. 20: in Schnittdarstellung den Blister gemäss der in Fig. 18 gezeigten Schnittebene B-B;
- Fig. 21: in Seitenansicht den Blister gemäss Fig. 18;
- Fig. 22: in perspektivischer Darstellung den Blister gemäss den Fig. 18 - 21;
- Fig. 23: in Draufsicht den Montageblock zum Montieren und Demontieren der Bohrstopphülsen auf den Dentalbohrer, wobei für die Montage der Montageblock für jeden Bohrdurchmesser der Dentalbohrer eine Bohrung aufweist, die geringfügig grösser ist als der Bohrdurchmesser des entsprechenden Dentalbohrers;
- Fig. 24: in Schnittdarstellung den Montageblock gemäss der in Fig. 23 eingezeichneten Schnittebene A-A;
- Fig. 25: in Seitenansicht den Montageblock gemäss Fig. 23 und 24, wobei diese Ansicht die Bohrungen zum Demontieren der Bohrstopphülsen von den Dentalbohrern zeigt;
- Fig. 26: in Schnittdarstellung den Montageblock gemäss der in Fig. 24 eingezeichneten Schnittebene B-B;
- Fig. 27: in perspektivischer Ansicht den Montageblock; und
- Fig. 28: in Seitenansicht eine weitere Ausführungsform eines Dentalbohrers, der im Vergleich zum in Fig. 1 gezeigten Dentalbohrer eine radiale Verdickung aufweist, in welche eine Nute zur Befestigung der Bohrstopphülse am Dentalbohrer ausgebildet ist.

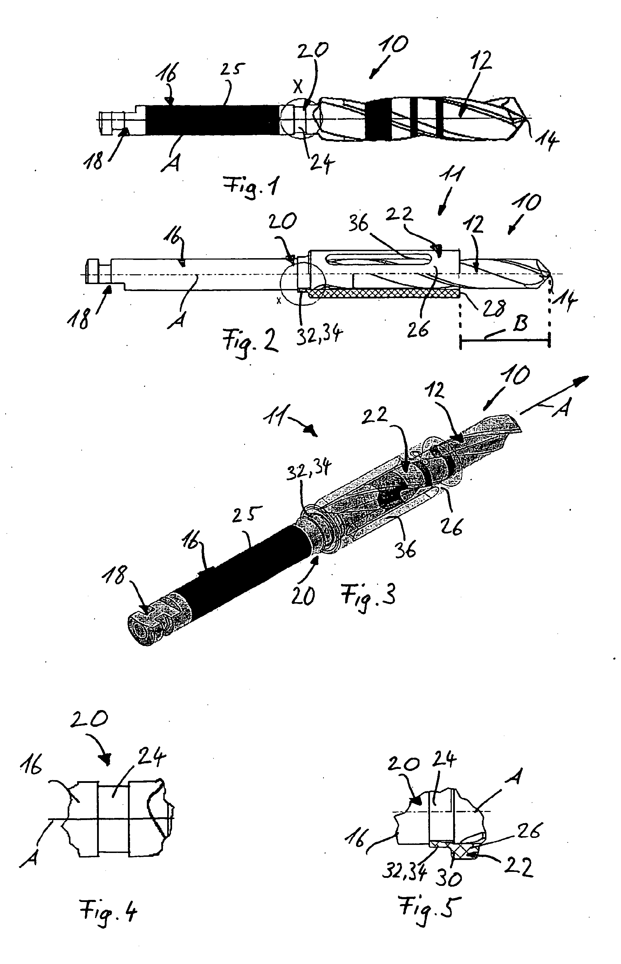

In Fig. 1 - 3 sind zwei Ausführungsbeispiele eines Dentalbohrers 10 gezeigt. Der Dentalbohrer 10 weist einerseits einen Schneidteil 12 mit einem stirnseitigen Bohrende 14 und andererseits einen Schaftteil 16 mit einem freiliegenden Aufnahmeendbereich 18 auf. Der Aufnahmeendbereich 18 ist dazu bestimmt, in einer allgemein bekannten Bohrerhaltevorrichtung aufgenommen zu werden und weist eine als Fläche ausgebildete Drehsicherung und eine als in Umgangsrichtung verlaufende Nute ausgebildete Axialsicherung auf. Durch die Drehsicherung und die Axialsicherung kann der Dentalbohrer 10 in eine feste Verbindung mit der Bohrerhaltevorrichtung gebracht werden, welche beispielsweise Bestandteil eines Bohrantriebs oder eines Handbohrers ist.

Der Schaftteil 16 weist, beabstandet vom Schneidteil 12, einen Haltebereich 20 für eine Bohrstopphülse 22 (siehe Fig. 2 und 3) auf. Wie insbesondere die Fig. 4 und 5 zeigen, ist in diesem Haltebereich 20 eine vollständig umlaufende Nute 24 in den Schaftteil 16 eingeformt. Diese Nute 24 weist im Wesentlichen zwei rechtwinklig zur Axialrichtung A des Dentalbohrers 10 stehende Nutenwände auf.

Wie in den Fig. 1 und 3 gezeigt, weist das eine Ausführungsbeispiel des Dentalbohrers 10 am Schaftteil 16, zwischen dem Aufnahmeendbereich 18 und dem Haltebereich 20, eine Farbkodierung 25 auf. Diese Farbkodierung ist am Dentalbohrer durch eine weitere in den Schaftteil eingeformte Nute ausgebildet, welche mit einem farbigen Material aufgefüllt ist. Als Material wird bevorzugt ABS-Kunststoff (Acrylnitril-Butadien-Styrol-Copolymer) verwendet. Die Farbe der Farbkodierung gibt dabei den Bohrdurchmesser des Dentalbohrers an. Bevorzugt werden Dentalbohrer mit Bohrdurchmessern von 2.2mm, 2.8mm, 3.5mm und 4.2mm verwendet. Selbstverständlich können die Dentalbohrer auch andere beziehungsweise weitere Bohrdurchmesser aufweisen. Bevorzugt weisen Dentalbohrer unterschiedlicher Bohrdurchmesser dieselbe Ausgestaltung des Aufnahmeendbereichs auf. Weiter weisen Dentalbohrer unterschiedlicher Bohrdurchmesser dieselbe Gesamtlänge sowie denselben Abstand der Nute 24 vom Bohrende auf.

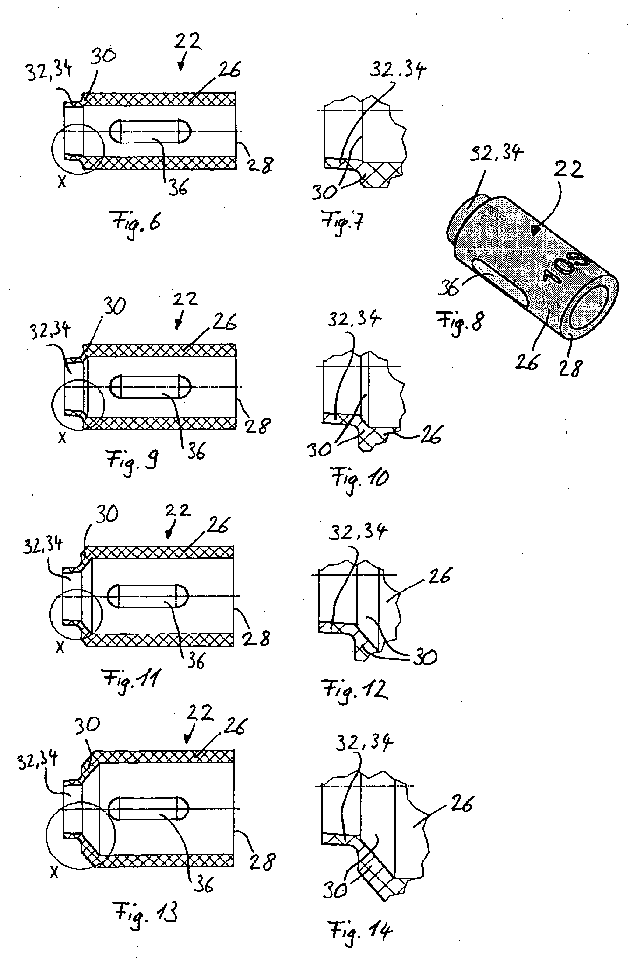

In den Fig. 2, 3 und 5 - 14 sind die Bohrstopphülsen 22 gezeigt, die dazu bestimmt sind, auf einen Dentalbohrer 10 aufgesteckt zu werden (siehe hierzu insbesondere Fig. 2, 3 und 5), um eine Bohrtiefe B zu begrenzen. Insbesondere zeigen die Fig. 6 - 14 Bohrstopphülsen 22 gleicher Länge, welche jedoch für Dentalbohrer 10 unterschiedlicher Bohrdurchmesser bestimmt sind.

Im Folgenden wird eine Dentalbohrvorrichtung beschrieben, welche durch einen Dentalbohrer 10 mit einem gegebenen Bohrdurchmesser und durch eine zu diesem Dentalbohrer 10 passende Bohrstopphülse 22 gegebener Länge gebildet ist.

Zum Begrenzen der Bohrtiefe B weist die Bohrstopphülse 22 einerseits, am freien Ende eines hohlzylinderförmigen Bereichs 26 eine Anschlagsfläche 28 auf. Die innere Mantelfläche des hohlzylinderförmigen Bereichs 26 ist dazu bestimmt, an den Schneidteil 12 des Dentalbohrers 10 anzuliegen und weist folglich einen Innendurchmesser auf, der im Wesentlichen dem Bohrdurchmesser des Dentalbohrers 10 entspricht. Daher wird dieser Innendurchmesser ebenfalls als Bohrdurchmesser oder als zum Bohrdurchmesser korrespondierender Hülsendurchmesser bezeichnet. Anschliessend an den hohlzylinderförmigen Bereich 26, auf der der Anschlagsfläche 28 gegenüberliegenden Seite, geht die Bohrstopphülse 22 in einen Befestigungsbereich 32 über. Im Befestigungsbereich 32 weist die Bohrstopphülse 22 eine ununterbrochen umlaufende Lippe 34 auf, die dazu bestimmt ist, in die Nute 24 im Haltebereich 20 des Dentalbohrers 10 einzugreifen. Eine freiliegende Stirnfläche der Lippe 34 wirkt dabei mit der dem Aufnahmeendbereich 18 zugewandeten Nutenwand zusammen. Die andere Nutenwand wirkt mit einer radial vorstehenden Anschlagfläche der Lippe 34 zusammen. Die auf den Dentalbohrer 10 aufgesetzte Bohrstopphülse 22 ist durch das Eingreifen der Lippe 34 in die Nute 24 in Axialrichtung A des Dentalbohrers 10 fixiert, wodurch die Bohrtiefe B, welche dem Abstand der Anschlagsfläche 28 zum Bohrende 14 des Dentalbohrers 10 entspricht, definiert ist.

Um die Bohrstopphülse 22 am Dentalbohrer 10 zu fixieren, wird diese vom Aufnahmeendbereich 18 her mit der Anschlagsfläche 28 voraus auf den Dentalbohrer 10 aufgeschoben. Die Lippe 34 wird dabei elastisch, in radialer Richtung ausgelenkt beziehungsweise in Umfangsrichtung elastisch gedehnt und schnappt, sobald beim Aufschieben der Bohrstopphülse 22 auf den Dentalbohrer 10 das freie Ende der Lippe 34 die Nute 24 erreicht, in diese ein und ist folglich in Axialrichtung A am Dentalbohrer gehalten.

Damit die elastische Verformung der Lippe 34 zuverlässig gewährleistet ist, weist die Bohrstopphülse 22 im Befestigungsbereich 32 eine wesentlich kleinere Wandstärke auf als im hohlzylinderförmigen Bereich 26. Im hohlzylinderförmigen Bereich 26 ist die Wandstärke konstant gewählt, insbesondere weist die Bohrstopphülse 22 ihren maximalen Aussendurchmesser im hohlzylinderförmigen Bereich 26 auf.

Selbstverständlich kann der Haltebereich 20 auch andersartig ausgebildet sein. Beispielsweise kann anstelle der Nute 24 der Haltebereich 20 eine Wulst aufweisen, die in Umfangsrichtung verläuft. Weiter kann die im Befestigungsbereich 32 der Bohrstopphülse 22 angeordnete Lippe 34 auch durch ein anderes Mittel zum Fixieren der Bohrstopphülse am Dentalbohrer ausgebildet sein. Wesentlich ist, dass der Befestigungsbereich 32 von der Anschlagfläche 28 beabstandet ist.

Im hohlzylinderförmigen Bereich 26 weist die Bohrstopphülse 22 zwei diametral gegenüberliegende radiale Durchgangsöffnungen 36 auf, welche in Axialrichtung A länglich ausgebildet sind. Diese Durchgangsöffnungen 36 sind dazu bestimmt, Material, welches beim Bohren aus dem Bohrloch gefördert wird, aus dem Inneren der Bohrstopphülse 22 auf die radial äussere Seite der Bohrstopphülse 22 zu fördern. Weiter dienen die radialen Durchgangsöffnungen 36 der Sicherstellung der Kühlung des Dentalbohrers 10 im Inneren der Bohrstopphülse 22.

Wie in den Fig. 6, 7 und 9 - 14 gezeigt, sind die Bohrstopphülsen an die oben genannten Bohrdurchmesser der Dentalbohrer 10 angepasst. Entsprechend den vier Bohrdurchmessern der Dentalbohrer 10 weisen die Bohrstopphülsen 22 vier verschiedene Hülsendurchmesser auf. Die vier in den oben genannten Figuren gezeigten Bohrstopphülsen 22 für verschiedene Bohrdurchmesser weisen jedoch dieselbe Länge auf, wodurch diese dieselbe Bohrtiefe für unterschiedliche Bohrdurchmesser definieren.

Der an den hohlzylinderförmigen Bereich 26 angrenzende Endbereich des Befestigungsbereich 32, als Zwischenbereich 30 bezeichnet, ist je nach Hülsendurchmesser der Bohrstopphülse 22 leicht unterschiedlich ausgebildet. Um einen stabilen Übergang von der Lippe 34 zum hohlzylinderförmigen Bereich 26 sicherzustellen, ist dieser Zwischenbereich insbesondere bei Bohrstopphülsen grösserer Hülsendurchmesser kegelstumpfartig ausgebildet.

Als Material für die Bohrstopphülsen 22 wird bevorzugt ein Kunststoff, insbesondere ein biokompatibler Kunststoff wie Polycarbonat verwendet. Beispielsweise kann das Produkt Makrolon (Produktname) der Firma Bayer AG verwendet werden. Um beim Bohren eine möglichst gute Sicht auf das Bohrloch zu gewährleisten, sind die Bohrstopphülsen 22 transparent oder zumindest transluszent. Dadurch ist während der ganzen Dauer des Bohrens, insbesondere kurz vor dem und beim Erreichen der durch die Dentalbohrvorrichtung festgelegten Bohrtiefe eine gute beziehungsweise zumindest eine verbesserte Sicht auf das Bohrloch gewährleistet. Dies ist bei Dentalbohrvorrichtungen gemäss dem Stand der Technik nicht gewährleistet, da dort beispielsweise Bohrstopphülsen aus Metall verwendet werden.

Weiter trägt jede der Bohrstopphülsen 22 eine der Kodierung des Dentalbohrers 10 entsprechende Kodierung, beispielsweise eine Farbkodierung, die den Innendurchmesser des hohlzylinderförmigen Bereichs 26 angibt. Dadurch kann der Anwender auf einfachste Weise erkennen, welche Bohrstopphülse 22 zu einem Dentalbohrer 10 mit einem gegebenen Bohrdurchmesser passt.

Weiter ist auf der Aussenseite jeder Bohrstopphülse 22 die Länge der Bohrstopphülse 22 angegeben beziehungsweise in geeigneter Art und Weise kodiert. Zusätzlich zur Länge oder anstelle der Länge der Bohrstopphülse 22 kann auch die durch die Länge der Bohrstopphülse 22 definierte Bohrtiefe B angegeben beziehungsweise kodiert sein.

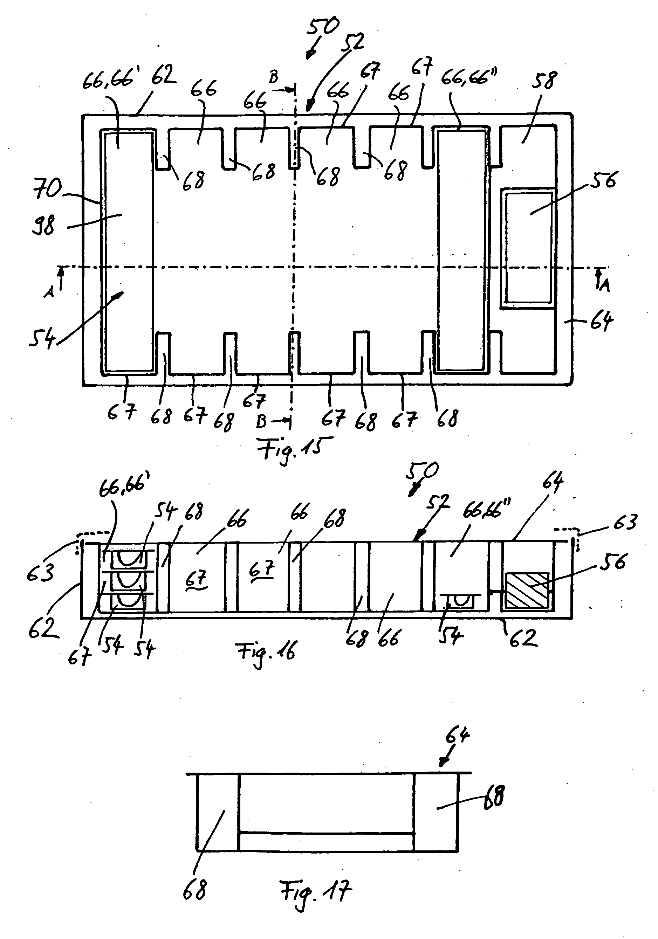

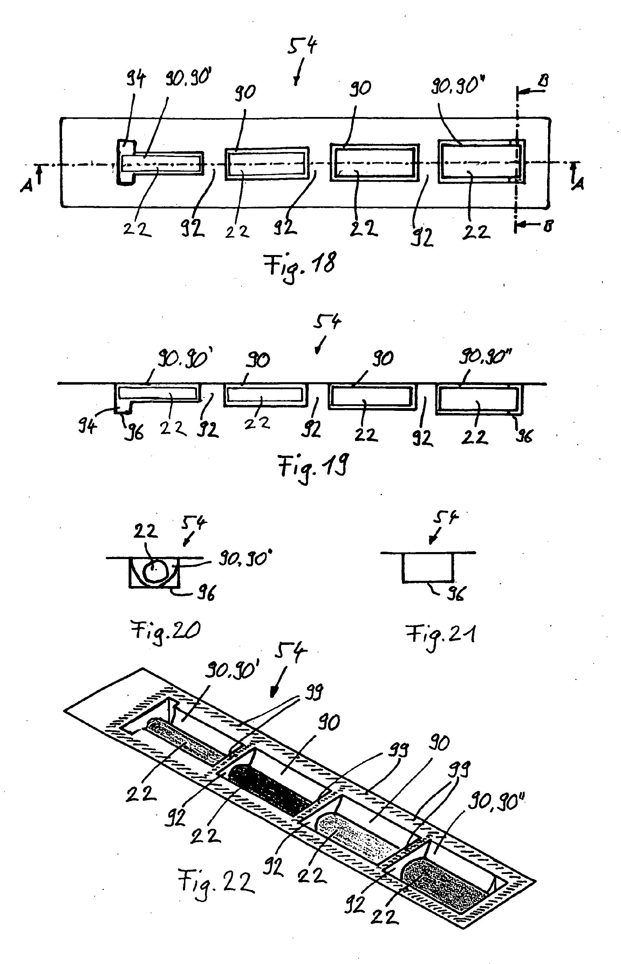

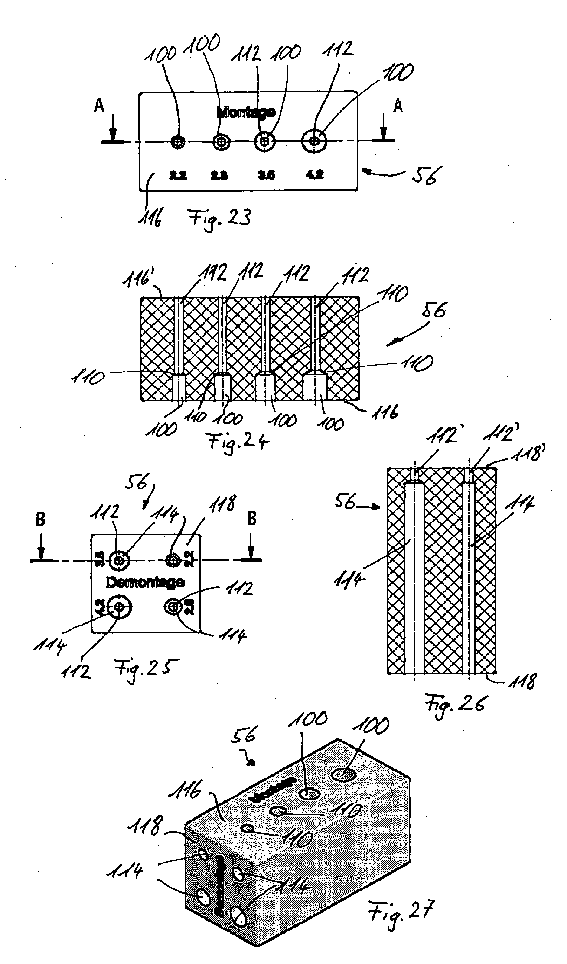

In den Fig. 15 - 17 ist ein Set 50 umfassend mehrer Bohrstopphülsen 22 verschiedener Längen und für Dentalbohrer 10 unterschiedlicher Bohrdurchmesser gezeigt. Weiter beinhaltet das Set 50 eine Setbox 52 mit darin gehaltenen Blistern 54 (siehe Fig. 18 - 22). Die Setbox 52 weist weiter eine Aufnahmevertiefung 58 für einen Beipackzettel und eine weitere für einen Montageblock 56 (siehe insbesondere Fig. 23 - 27) auf. Mittels des Montageblocks 56 ist die Montage und die Demontage der Bohrstopphülsen 22 auf die Dentalbohrer 10 beziehungsweise von den Dentalbohrern 10 auf einfachste Weise durchführbar.

Die in Fig. 15 - 17 gezeigte Setbox 50 weist eine einen Schachtelunterteil bildende Aussenhülle 62 auf, auf welche ein Deckel 63 (in Fig. 16 durch punktierte Linie angedeutet) in bekannter Art und Weise aufsetzbar ist. Dieser Deckel 63 ist bevorzugt aus einem transparenten und die Aussenhülle 62 aus einem nicht transparenten Material gefertigt. In die Aussenhülle 62 ist ein bevorzugt dünnwandiger, aus Kunststoff gefertigter Boxeneinsatz 64 eingesetzt. Dieser dient dazu, sechs Blister 54 nebeneinander und jeweils drei übereinander zu halten sowie den Montageblock 56 und den Beipackzettel aufzunehmen. Selbstverständlich kann der Boxeneinsatz 64 auch derart ausgebildet sein, dass eine andere Anzahl von Blistern 54 nebeneinander beziehungsweise übereinander angeordnet werden könne.

Um die Blister 54 im Boxeinsatz 64 nebeneinander geordnet zu halten, weist der Boxeneinsatz 64 mehrere, integral ausgebildete Blisteraufnahmevertiefungen 66 auf, von denen jede drei Blister 54 übereinander aufnehmen kann. Die Blisteraufnahmevertiefungen 66 sind derart nebeneinander anordnet, dass ihre Längsseiten nebeneinander liegen. In Längsrichtung der Blisteraufnahmevertiefungen 66 sind diese durch Seitenwände 67 des Boxeinsatzes 64 begrenzt. Um die Blisteraufnahmevertiefungen auch in ihrer Querrichtung zu begrenzen, weisen die Seitenwänden 67 jeweils zwischen zwei nebeneinander liegenden Blisteraufnahmevertiefungen 66 ein vorstehendes, stegartiges Separierelement 68 auf. Die eine äusserste Blisteraufnahmevertiefung 66' ist weiter durch eine die Seitenwände 67 miteinander verbindende Querwand 70 des Boxeinsatzes 64 begrenzt und die andere äusserste Blisteraufnahmevertiefung 66" ist durch ein weiteres Paar von Separierelementen 68 begrenzt, welche diese Blisteraufnahmevertiefung 66" von der ebenfalls integral ausgebildeten Aufnahmevertiefung 58 für den Beipackzettel abgrenzt.

Integral mit der Aufnahmevertiefung 58 für den Beipackzettel ist die Aufnahmevertiefung für den Montageblock 56 ausgebildet, wobei diese mittig, nach unten sich von der Aufnahmevertiefung für den Beipackzettel 58 fortsetzt.

Der in Fig. 18 - 22 gezeigte Blister 54 weist vier Aufnahmevertiefungen 90 - analog zu den vier Bohrdurchmessern der Dentalbohrer 10 - auf, die in Längsrichtung des Blisters 54 hintereinander angeordnet sind. Die Aufnahmevertiefungen 90 sind je durch eine Zwischenwand 92 voneinander getrennt. In jeder Aufnahmevertiefung 90 ist eine Bohrstopphülse 22 eingelegt, deren Längsachse in Längsrichtung des Blisters 54 verläuft. Im Blister 54 sind vier Bohrstopphülsen 22 für die vier unterschiedlichen Bohrdurchmesser jedoch nur für eine Bohrtiefe gehalten. Die vier Aufnahmevertiefungen 90 weisen alle dieselbe Länge auf, jedoch sind die Tiefen und Breiten der jeweiligen in der Aufnahmevertiefung 90 aufgenommenen Bohrstopphülse 22 angepasst, insbesondere ist die Tiefe derart angepasst, dass die Bohrstopphülse 22 knapp unterhalb der oberen Kante der Aufnahmevertiefung 90 liegt. Mit anderen Worten ist die Aufnahmevertiefung 90 etwas tiefer als der Aussendurchmesser der in der Aufnahmevertiefung 90 aufgenommenen Bohrstopphülse 22. Die Anordnung der Aufnahmevertiefungen 90 ist derart gewählt, dass in Längsrichtung des Blisters 54 die Bohrstopphülsen 22 nach zunehmenden Bohrdurchmessern geordnet sind.

Weiter ist aus Fig. 22 gut erkennbar, dass die im Blister 54 aufgenommenen Bohrstopphülsen 22 farbkodiert sind. Diese können beispielsweise die Farben Blau, Gelb, Rot und Grün aufweisen.

Damit die Blister 54 problemlos stapelbar sind, weist die Aufnahmevertiefung 90' für die Bohrstopphülse 22 mit dem geringsten Bohrdurchmesser einen nach unten vorstehenden Fortsatz 94 auf, sodass die maximale Tiefe dieser Aufnahmevertiefung gleich ist, wie die Tiefe der Aufnahmevertiefung für die Bohrstopphülse mit dem grössten Bohrdurchmesser. Weiter bildet dieser Fortsatz 94 auf der äusseren, nach unten ausgerichteten Seite eine Fläche 96 aus. Durch diese Fläche 96 und einer weiteren, analogen, an der Aufnahmevertiefung 90" für die Bohrstopphülse 22 mit dem grössten Bohrdurchmesser ausgebildeten Fläche 96 wird erreicht, dass beim Aufsetzen des Blisters 54 auf eine horizontale Ebene die Oberseite des Blisters 54 ebenfalls horizontal ausgerichtet ist.

Jede Aufnahmevertiefung 90 ist mittels eines Abziehsteifens 98 (siehe Fig. 15) steril verschlossen, der sich über die gesamte Länge des Blisters erstreckt. Hierzu ist jede Aufnahmevertiefung 90 durch einen die Aufnahmevertiefung 90 umlaufende Siegelnahtbereich 99 (siehe Fig. 22) umgeben, in welchem der Abziehstreifen 98 durch Versiegeln fest mit dem Blister 54 verbunden wird. Dadurch ist auf einfachste Weise eine sterile Verpackung für jede einzelne Bohrstopphülse 22 erreichbar.

Der in den Fig. 23 - 27 gezeigte, quaderförmige Montageblock 56 ist zum Montieren der Bohrstopphülsen 22 auf und zum Demontieren der Bohrstopphülsen 22 von den Dentalbohrern 10 bestimmt und weist vier Längsseitenflächen 116 und zwei Stirnflächen 118 auf. Für die Montage weist der Montageblock 56 für jeden der vier Bohrdurchmesser der Dentalbohrer 10 eine Montagevertiefung 100 auf, die von einer der Längsseitenflächen 116 in den Montageblock 56 hinein führt und deren Durchmesser geringfügig grösser ist als der Bohrdurchmesser des jeweiligen Dentalbohrers 10. Jede der Montagevertiefungen 100 ist stirnseitig durch einen Boden 110 begrenzt, der für einen in die Montagevertiefung 100 eingeführten Dentalbohrer einen Anschlag bildet. Die durch den Boden 110 definierte Tiefe der Montagevertiefung 100 ist derart gewählt, dass diese kleiner ist als die durch die längste Bohrstopphülse definierte minimale Bohrtiefe. Wie Fig. 23 und 27 zeigt, sind die vier Montagevertiefungen 100 entlang einer Mittellinie angeordnet. Weiter weist jede Montagevertiefung 100 ein in Axialrichtung der Montagevertiefung 100 verlaufendes Durchgangsloch 112 auf, welches die Reinigung der Montagevertiefungen 100 erleichtert. Der Durchmesser der Durchgangslochs 112 ist kleiner gewählt als der kleinste Bohrdurchmesser. Dieses Durchgangsloch 112 mündet einerseits mittig in den Boden 110 und andererseits in der gegenüberliegenden Längsseitenfläche 116' (siehe Fig. 24).

Zur Demontage weist der Montageblock 56 vier Demontagevertiefungen 114 auf, die derart angeordnet sind, dass die Demontagevertiefungen 114 nicht mit den Montagevertiefungen 100 zusammen treffen. Die Demontagevertiefungen 114 führen von einer der beiden Stirnflächen 118 in den Montageblock hinein. Die Demontagevertiefungen 114 sind weitgehend analog zu den Montagevertiefungen 100 ausgebildet, weisen jedoch eine Tiefe auf, die grösser ist als die durch die kürzeste Bohrstopphülse 22 definierte maximale Bohrtiefe. Vom Boden 110 jeder Demontagevertiefung 114 führt in Axialrichtung der Demontagevertiefung 114 ein Durchgangsloch 112' zu der der Öffnung der Demontagevertiefung 114 gegenüberliegenden Stirnfläche 118'.

Die Bohrstopphülse 22, die Dentalbohrvorrichtung 11 und das Set 50 werden wie folgt verwendet:

Um beispielsweise ein Bohrloch vorgegebener Lochtiefe und vorgegebenem Lochdurchmesser in den Kiefer eines Patienten zu bohren wird vielfach zunächst mittels eines Dentalbohrers 10 ein Bohrloch der gewünschten Länge, jedoch mit einem kleineren Bohrdurchmesser gebohrt. Darauffolgend wird dieses Bohrloch sodann in einem oder mehreren Schritten mit Dentalbohrern 10 grösserer Bohrdurchmesser auf den gewünschten Lochdurchmesser aufgebohrt.

Um sicherzustellen, dass das Bohrloch die gewünschte Lochtiefe aufweisen wird, wird die Bohrtiefe des Dentalbohrers durch Verwendung einer Bohrstopphülse entsprechender Länge begrenzt. Da Bohrstopphülsen 22 gleicher Längen für die unterschiedlichen Bohrdurchmesser in demselben Blister 54 aufbewahrt werden, wird vor der Operation der Blister 54 mit den Bohrstopphülsen 22 der gewünschten Länge der Setbox 50 entnommen. Da ein Blister 54 nur Bohrstopphülsen 22 gleicher Länge aufweist, ist ein versehentliches Verwechseln von Bohrstopphülsen 22 verschiedener Längen ausgeschlossen. Folglich kann durch das erfindungsgemässe Set und durch die erfindungsgemässe Dentalbohrvorrichtung 11 beispielsweise eine Verletzung von im Kiefer verlaufenden Nerven zuverlässig verhindert werden.

Darauffolgend werden auf die Dentalbohrer 10 mit den gewünschten Bohrdurchmessern die dazu passenden Bohrstopphülsen 22 montiert, wobei durch die Kodierung klar gekennzeichnet ist, welche Bohrstopphülse 22 auf welchen Dentalbohrer 10 aufzusetzen ist.

Zum Aufsetzen der Bohrstopphülsen 22 auf die Dentalbohrer 10 werden diese mit dem Bohrende 14 voraus in die entsprechenden Montagevertiefungen 100 des Montageblocks 56 gesteckt, wodurch zum einen der scharfe, empfindliche Schneidteil 12 vor einer Beschädigung während der Montage geschützt ist und zum anderen eine Verletzungsgefahr für diejenige Person vermindert ist, welche die Montage durchführt. Darauffolgend werden die entsprechenden Bohrstopphülsen 22 mit der Anschlagfläche 28 voraus auf die Dentalbohrer 10 von dem Aufnahmeendbereich 18 her aufgeschoben, bis die Lippe 34 jeder Bohrstopphülse 22 in die Nute 24 am Schaftteil 16 des entsprechenden Dentalbohrers 10 einschnappt.

Falls eine Bohrstopphülse 22 nach dem Bohren des Bohrlochs vom Dentalbohrer 10 zu entfernen ist, da beispielsweise der Dentalbohrer für ein weiteres Bohrloch unterschiedlicher Länge verwendet werden soll, wird der Dentalbohrer 10 mit dem Bohrende 14 voraus in die dem Bohrdurchmesser entsprechende Demontagevertiefung 114 des Montageblocks 56 eingeführt bis die Anschlagfläche 28 der Bohrstopphülse 22 am Montageblock 56 ansteht. Durch nachfolgendes Hineindrücken des Dentalbohrers 10 in die Demontagevertiefung 114 wird sodann die durch das Eingreifen der Lippe 34 der Bohrstopphülse 22 in die Nute 24 des Dentalbohrers 10 gelöst und die Bohrstopphülse 22 kann vom Dentalbohrer 10 entfernt werden. Wiederum kann durch die Verwendung des Montageblocks 56 zur Demontage der Bohrstopphülse 22 eine Beschädigung des Dentalbohrers 10 vermieden beziehungsweise eine Verletzungsgefahr für diejenige Person vermindert werden, welche die Demontage durchführt.

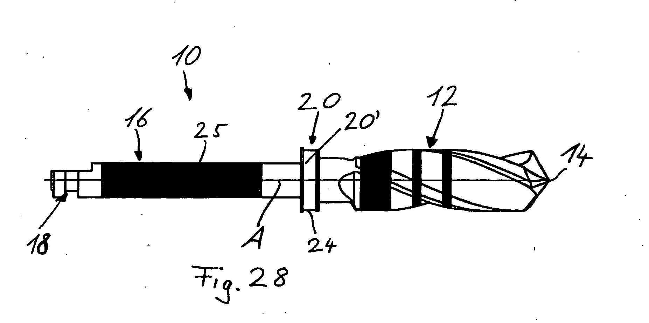

Eine weitere Ausführungsform des Dentalbohrers 10 ist in Fig. 28 gezeigt. Dieser Dentalbohrer 22 weist - im Unterschied zu den in Fig. 1 bis 3 gezeigten Dentalbohrern 10 - im Haltebereich 20 eine radiale Verdickung 20' des Schaftteils 16 auf, in welche eine Nute 24 eingeformt ist. Die beiden Nutenwände sind durch kreisscheibenförmige Abschnitte im Haltebereich 20 ausgebildet, wobei der dem Aufnahmeendbereich 18 zugewandte kreischeibenförmige Abschnitt den grösseren Durchmesser aufweist als der dem Bohrende 14 zugewandte Abschnitt. Der dem Bohrende 14 zugewandte kreischeibenförmige Abschnitt weist einen Durchmesser auf, der im Wesentlichen gleich dem Bohrdurchmesser des Schneidteils 12 ist.

Eine zu dem in Fig. 28 gezeigten Dentalbohrer 10 passende Bohrstopphülse weist im Wesentlichen dieselbe Form und Eigenschaften auf wie die im Zusammenhang mit Fig. 6 bis 8 beschriebene Bohrstopphülse 22, wird jedoch mit dem Befestigungsbereich 32 voraus auf den Dentalbohrer 10 von dessen Bohrende 14 her aufgeschoben. Die Lippe 34 der Bohrstopphülse 22 ist wiederum dazu bestimmt, in die Nute 24 im Haltebereich 20 einzugreifen.

Eine weitere Ausführungsform der Bohrstopphülse kann über ihre gesamte Länge im Wesentlichen zylinderförmig ausgebildet sein. Im Befestigungsbereich weist eine solche Bohrstopphülse eine in ihre radial innenliegende Mantelfläche eingeformte, radial vollständig umlaufende Nute auf, die mit dem dem Bohrende 14 zugewandten kreischeibenförmigen Abschnitt zur Befestigung der Bohrstopphülse am Dentalbohrer 10 zusammen wirkt. Der dem Aufnahmeendbereich 18 zugewandte kreischeibenförmige Abschnitt wirkt als Anschlag mit einer Stirnfläche des Befestigungsbereichs der Bohrstopphülse zusammen.

Der Befestigungsbereich einer für den Dentalbohrer gemäss Fig. 28 bestimmten Bohrstopphülse ist wiederum elastisch deformierbar. Für weitere Eigenschaften und Material wird auf die oben, in Zusammenhang mit Fig. 6 bis 8 beschriebenen Bohrstopphülsen verwiesen.

Die oben beschriebenen Dentalbohrer 10 und Bohrstopphülsen 22 können derart ausgelegt sein, dass sie nur für eine einzige Operation verwendet werden. Da folglich auf eine Sterilisation nach Verwendung der verwendeten Dentalbohrer 10 und Bohrstopphülsen 22 verzichtet werden kann, können Materialien, insbesondere Kunststoffe verwenden werden, die nur schwierig sterilisiert werden können und im Verglich zu sterilisierbaren Kunststoffen vergleichsweise kostengünstig sind.

## Patentansprüche

1. Set beinhaltend mehrere, zum Aufsetzen auf Dentalbohrer (10) unterschiedlicher Bohrdurchmesser bestimmte Bohrstopphülsen (22) unterschiedlicher Längen und unterschiedlicher, zu den Bohrdurchmessern korrespondierenden Hülsendurchmessern, wobei die Bohrstopphülsen (22) in Blistern (54) gehalten sind, die für jede Bohrstopphülse (22) eine separate Aufnahmevertiefung (90) aufweisen, jeder Blister (54) mehrere Bohrstopphülsen (22) gleicher Länge und von jedem Hülsendurchmesser eine Bohrstopphülse (22) aufweist, **dadurch gekennzeichnet, dass** das Set (50) einen Montageblock (56) zum Montieren und Demontieren der Bohrstopphülsen (22) auf die beziehungsweise von den Dentalbohrern (10) aufweist, wobei der Montageblock (56) für Dentalbohrer (10) jeden Bohrdurchmessers eine kreiszylinderförmige Montagevertiefung (100) mit einem geringfügig grösseren Durchmesser als der Bohrdurchmesser aufweist und stirnseitig durch einen Anschlag für den Dentalbohrer bildenden Boden (110) begrenzt ist, wobei die Tiefe der Montagevertiefung (100) kleiner ist als die minimale Bohrtiefe, welche durch den Abstand der Anschlagfläche (28) der am Dentalbohrer (10) montierten längsten Bohrstopphülse (22) vom Bohrende (14) des Dentalbohrers (10) definiert ist, und für Dentalbohrer (10) jeden Bohrdurchmessers eine kreiszylinderförmige Demontagevertiefung (114) mit einem geringfügig grösseren Durchmesser als der Bohrdurchmesser aufweist, deren Tiefe grösser ist als die maximale Bohrtiefe, welche durch den Abstand der Anschlagfläche (28) der am Dentalbohrer (10) montierten kürzesten Bohrstopphülse (22) vom Bohrende (14) des Dentalbohrers (10) definiert ist.

2. Set nach Anspruch 1, **dadurch gekennzeichnet, dass** Blister (54) beinhaltend Bohrstopphülsen (22) unterschiedlicher Längen nebeneinander und Blister (54) beinhaltend Bohrstopphülsen (22) gleicher Längen übereinander in einer Setbox (52) angeordnet sind.

3. Set nach Anspruch 1 oder 2, **dadurch gekennzeichnet, dass** die Bohrstopphülse aus Kunststoff gefertigt und auf den Dentalbohrer (10) aufsetzbar ist und stirnseitig, an einem freien Ende eines hohlzylinderförmigen Bereichs (26) eine Anschlagfläche (28) und in einem von dieser beabstandeten Befestigungsbereich (32) ein Mittel zum Fixieren (34) der Bohrstopphülse (22) am Dentalbohrer (10) aufweist und dass die Bohrstopphülse (22) transparent oder transluzent ist.

4. Set gemäss Anspruch 3, **dadurch gekennzeichnet, dass** der Befestigungsbereich (32) beim der Anschlagfläche (28) gegenüberliegenden Ende der Bohrstopphülse (22) angeordnet ist.

5. Set gemäss Anspruch 3 oder 4, **gekennzeichnet durch** eine in Axialrichtung (A) der Bohrstopphülse (22) in etwa mittig angeordnete, radiale Durchgangsöffnung (36) im holzylinderförmigen Bereich (26).

6. Set gemäss einem der Ansprüche 3 bis 5, **dadurch gekennzeichnet, dass** die Bohrstopphülse im hohlzylinderförmigen Bereich (26), die Wandstärke grösser ist als die Wandstärke im Befestigungsbereich (32), und der Innendurchmesser im Befestigungsbereich (32) sich in Richtung des freien Endes verjüngt.

7. Set nach einem der Ansprüche 3 bis 6, **dadurch gekennzeichnet, dass** der Dentalbohrer (10) einerseits einen Schneidteil (12) mit einem stirnseitigen Bohrende (14) und andererseits einen Schaftteil (16) mit einem Aufnahmeendbereich (18) aufweist, welcher dazu bestimmt ist, in einer Bohrerhaltevorrichtung aufgenommen zu werden, und wobei am Schaftteil (16) eine Nute (24) oder ein Wulst in einem Haltebereich (20) ausgebildet ist, welcher mit dem Befestigungsbereich (32) der Bohrstopphülse (22) derart zusammenwirkt, dass dadurch eine Bohrtiefe (B) durch den Abstand vom Bohrende (14) zur Anschlagfläche (28) der Bohrstopphülse definiert ist, wobei die Bohrstopphülse (22) im Befestigungsbereich (32) eine ununterbrochen umlaufende Lippe (34) aufweist, welche mit der Nute (24) beziehungsweise der Wulst zusammen wirkt.

8. Set nach Anspruch 7, **dadurch gekennzeichnet, dass** der Haltebereich (20) eine vollständig umlaufende, in den Schaftteil (16) eingelassene. Nute (24) aufweist, in welche die Lippe (34) eingreift.

9. Set nach Anspruch 7 oder 8, **dadurch gekennzeichnet, dass** die Lippe (34) derart elastisch deformierbar ist, dass die Bohrstopphülse (22) über den Schaftteil (16) auf den Dentalbohrer (10) aufsetzbar ist.

10. Set nach einem der Ansprüche 3 bis 9, **dadurch gekennzeichnet, dass** die Bohrstopphülse (22) und der Dentalbohrer (10) eine Kodierung, insbesondere eine Farbkodierung (25), zur Kennzeichnung des Innendurchmessers des hohlzylinderförmigen Bereichs (26) beziehungsweise des Bohrdurchmessers des Dentalbohrers (10) aufweisen.

## Claims

1. Set containing several drill stop sleeves (22) which are designed to be fitted onto dental drills (10) of different bore diameters and which have different lengths and different sleeve diameters corresponding to the bore diameters, the drill stop sleeves (22) being held in blisters (54) that have a separate receiving recess (90) for each drill stop sleeve (22), each blister (54) comprising several drill stop sleeves (22) of equal length and a drill stop sleeve (22) of each sleeve diameter, **characterized in that** the set (50) has an assembly block (56) for assembly and disassembly of the drill stop sleeves (22) onto and from the dental drills (10), the assembly block (56) having, for dental drills (10) of each bore diameter, a circular cylindrical assembly recess (100) with a slightly greater diameter than the bore diameter and limited at the end by a base (110) that forms a limit stop for the dental drill, the depth of the assembly recess (100) being smaller than the minimum drilling depth, which is defined by the distance of the abutment surface (28) of the longest drill stop sleeve (22) mounted on the dental drill (10) from the drill end (14) of the dental drill (10), and having, for dental drills (10) of each bore diameter, a circular cylindrical disassembly recess (114) with a slightly greater diameter than the bore diameter and whose depth is greater than the maximum drilling depth, which is defined by the distance of the abutment surface (28) of the shortest drill stop sleeve (22) mounted on the dental drill (10) from the drill end (14) of the dental drill (10).

2. Set according to Claim 1, **characterized in that** blisters (54) containing drill stop sleeves (22) of different lengths are arranged next to one another, and blisters (54) containing drill stop sleeves (22) of equal lengths are arranged above one another in a set box (52).

3. Set according to Claim 1 or 2, **characterized in that** the drill stop sleeve is made of plastic and can be fitted onto the dental drill (10), has an abutment surface (28) at a free end of a hollow cylindrical area (26) and, in a securing area (32) at a distance from the abutment surface (28), has a means (34) for fixing the drill stop sleeve (22) on the dental drill (10), and that the drill stop sleeve (22) is transparent or translucent.

4. Set according to Claim 3, **characterized in that** the securing area (32) is arranged at the end of the drill stop sleeve (22) opposite to the abutment surface (28).

5. Set according to Claim 3 or 4, **characterized by** a radial through-opening (36) in the hollow cylindrical area (26), which radial through-opening (36) is arranged approximately centrally in the axial direction (A) of the drill stop sleeve (22).

6. Set according to one of Claims 3 to 5,
**characterized in that**, in the hollow cylindrical area (26) of the drill stop sleeve, the wall thickness is greater than the wall thickness in the securing area (32), and the internal diameter in the securing area (32) narrows in the direction of the free end.

7. Set according to one of Claims 3 to 6, **characterized in that** the dental drill (10) on the one hand having a cutting part (12) with a drilling end (14) and on the other hand having a shank part (16) with a receiving end area (18) designed to be received in a drill-holder device, and a groove (24) being formed on the shank part (16) or a bead being formed in a holding area (20), which interacts with the securing area (32) of the drill stop sleeve (22) in such a way that a drilling depth (B) is thus defined by the distance from the drilling end (14) to the abutment surface (28) of the drill stop sleeve, wherein the drill stop sleeve (22), in the securing area (32), having an uninterrupted circumferential lip (34) that interacts with the groove (24) or bead, respectively.

8. Set according to Claim 7, **characterized in that** the holding area (20) comprises a complete circumferential groove (24), which is worked into the shank part (16) and into which the lip (34) engages.

9. Set according to Claim 7 or 8, **characterized in that** the lip (34) is elastically deformable in such a way that the drill stop sleeve (22) can be fitted over the shank part (16) onto the dental drill (10).

10. Set according to one of Claims 3 to 9, **characterized in that** the drill stop sleeve (22) and the dental drill (10) have a coding, in particular a color coding (25), for identifying the internal diameter of the hollow cylindrical area (26) or the bore diameter of the dental drill (10).

## Revendications

1. Set contenant plusieurs douilles pour limiter le forage (22) prévues pour être posées sur des forets dentaires (10) de différents diamètres de forage, ayant des longueurs différentes et des diamètres de douille différents, correspondant aux diamètres de forage, les douilles pour limiter le forage (22) étant retenues dans des coques (54), qui présentent une cavité de réception séparée (90) pour chaque douille pour limiter le forage (22), chaque coque (54) présentant plusieurs douilles pour limiter le forage (22) de même longueur, et une douille pour limiter le forage (22) de chaque diamètre de douille,
**caractérisé en ce que** le set (50) présente un bloc de montage (56) pour le montage des douilles pour limiter le forage (22) sur les forets dentaires (10) ou pour le démontage des forets dentaires, le bloc de montage (56) pour des forets dentaires (10) de chaque diamètre de forage présentant une cavité de montage (100) de forme cylindrique circulaire avec un diamètre légèrement plus grand que le diamètre de forage, et étant limité du côté frontal par un fond (110) formant une butée pour le foret dentaire, la profondeur de la cavité de montage (100) étant inférieure à la profondeur minimale de forage, qui est définie par la distance de la surface de butée (28) de la douille pour limiter le forage (22) la plus longue montée sur le foret dentaire (10) depuis l'extrémité de forage (14) du foret dentaire (10), et présentant une cavité de démontage (114) de forme cylindrique circulaire pour des forets dentaires (10) de chaque diamètre de forage, avec un diamètre légèrement plus grand que le diamètre de forage, dont la profondeur est plus grande que la profondeur maximale de forage, qui est définie par la distance de la surface de butée (28) de la douille pour limiter le forage (22) la plus courte montée sur le foret dentaire (10) depuis l'extrémité de forage (14) du foret dentaire (10).

2. Set selon la revendication 1, **caractérisé en ce que** des coques (54) contenant des douilles pour limiter le forage (22) de différentes longueurs sont disposées les unes à côté des autres et des coques (54) contenant des douilles pour limiter le forage (22) de même longueur sont disposées les unes au-dessus des autres dans une boîte de set (52).

3. Set selon la revendication 1 ou 2, **caractérisé en ce que** la douille pour limiter le forage est en plastique et peut être posée sur le foret dentaire (10) et présente, du côté frontal, à une extrémité libre d'une région de forme cylindrique creuse (26), une surface de butée (28), et dans une région de fixation (32) espacée de celle-ci un moyen de fixation (34) de la douille pour limiter le forage (22) sur le foret dentaire (10), et **en ce que** la douille pour limiter le forage (22) est transparente ou translucide.

4. Set selon la revendication 3, **caractérisé en ce que** la région de fixation (32) est disposée au niveau de l'extrémité de la douille pour limiter le forage (22) opposée à la surface de butée (28).

5. Set selon la revendication 3 ou 4, **caractérisé par** une ouverture de passage (36) radiale, disposée approximativement centralement dans la direction axiale (A) de la douille pour limiter le forage (22), dans la région de forme cylindrique creuse (26).

6. Set selon l'une quelconque des revendications 3 à 5, **caractérisé en ce que** la douille pour limiter le forage a dans la région de forme cylindrique creuse (26) une plus grande épaisseur de paroi que dans la région de fixation (32), et le diamètre intérieur dans la région de fixation (32) diminue dans la direction de l'extrémité libre.

7. Set selon l'une quelconque des revendications 3 à 6, **caractérisé en ce que** le foret dentaire (10) présente d'une part une partie coupante (12) avec une extrémité de forage frontale (14) et d'autre part une partie de tige (16) avec une région d'extrémité de réception (18), qui est prévue pour être reçue dans un dispositif de retenue de foret, une rainure (24) ou un bourrelet étant réalisé(e) dans une région de retenue (20) sur la partie de tige (16), afin de coopérer avec la région de fixation (32) de la douille pour limiter le forage (22), de telle sorte qu'une profondeur de forage (B) soit définie par la distance de l'extrémité de forage (14) à la surface de butée (28) de la douille pour limiter le forage, la douille pour limiter le forage (22) présentant dans la région de fixation (32) une lèvre périphérique ininterrompue (34) qui coopère avec la rainure (24) ou le bourrelet.

8. Set selon la revendication 7, **caractérisé en ce que** la région de retenue (20) présente une rainure (24) pratiquée dans la partie de tige (16) sur toute la périphérie, dans laquelle s'engage la lèvre (34).

9. Set selon la revendication 7 ou 8, **caractérisé en ce que** la lèvre (34) peut être déformée élastiquement de telle sorte que la douille pour limiter le forage (22) puisse être posée pardessus la partie de tige (16) sur le foret dentaire (10).

10. Set selon l'une quelconque des revendications 3 à 9, **caractérisé en ce que** la douille pour limiter le forage (22) et le foret dentaire (10) présentent un codage, notamment un codage de couleur (25), pour identifier le diamètre intérieur de la région (26) de forme cylindrique creuse ou le diamètre de forage du foret dentaire (10).
